(19) Europäisches Patentamt
European Patent Office
Office européen des brevets

(11) EP 2 316 411 B1

(12) EUROPEAN PATENT SPECIFICATION

(45) Date of publication and mention of the grant of the patent:
**12.02.2014 Bulletin 2014/07**

(51) Int Cl.:
***A61K 8/34*** *(2006.01)* ***A61K 8/41*** *(2006.01)*
***A61Q 19/08*** *(2006.01)*

(21) Application number: **10251712.5**

(22) Date of filing: **01.10.2010**

# (54) Compositions comprising a skin-lightening resorcinol and a skin darkening agent

Zusammensetzungen mit hautaufhellendem Resorcinol und Hauttönungsmittel

Compositions comprenant du résorcinol pour éclaircir la peau et un agent pour foncer la peau

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.10.2009 US 572650**

(43) Date of publication of application:
**04.05.2011 Bulletin 2011/18**

(73) Proprietor: **Johnson & Johnson Consumer Companies, Inc.**
**Skillman, NJ 08558 (US)**

(72) Inventors:
- **Cochran, Steven**
  **Princeton, NJ 08540 (US)**
- **Kizoulis, Menas G.**
  **Bound Brook, NJ 08805 (US)**
- **Southall, Michael**
  **Lawrenceville, NJ 08648 (US)**
- **Tyerech, Michael**
  **Ringoes, NJ 08551 (US)**

(74) Representative: **Kirsch, Susan Edith**
**Carpmaels & Ransford LLP**
**One Southampton Row**
**London WC1B 5HA (GB)**

(56) References cited:
**EP-A2- 1 250 908** **EP-A2- 1 974 773**
**EP-A2- 2 045 297** **WO-A2-03/082231**
**DE-A1- 10 118 894**

- **DATABASE WPI Week 200930 Thomson Scientific, London, GB; AN 2009-H70662 XP002635472, & JP 2009 084164 A (SEPTEM SOKEN KK) 23 April 2009 (2009-04-23)**
- **DATABASE WPI Week 200849 Thomson Scientific, London, GB; AN 2008-H65100 XP002635473, & JP 2007 254412 A (KURARAY CO LTD) 4 October 2007 (2007-10-04)**
- **DATABASE WPI Week 200223 Thomson Scientific, London, GB; AN 2002-174418 XP002635474, & JP 2001 302505 A (KURARAY CO LTD) 31 October 2001 (2001-10-31)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### FIELD OF THE INVENTION

A composition comprising a skin-lightening resorcinol and a skin darkening agent as described in claim 1 is provided. The composition is useful, for example, for topical application to the skin.

### BACKGROUND OF THE INVENTION

A vast array of cosmetic agents are known to aid in modifying the appearance of one's skin. The effect can be direct and immediate. For example, the agent may be a simple reflectant or pigment (such as those in "make-up" or "foundation") that is applied to the surface of the skin, thereby causing skin to appear more pleasant and even. Another example of agents capable of providing virtually immediate changes in appearance are "so-called" tensioning polymers. Tensioning polymers act by pulling the skin tight as they dry, thereby reducing the appearance of wrinkles, a common sign of aging.

Changes in appearance can also be less direct and develop over time. For example, sunscreens, by screening out ultraviolet light, can indirectly assist in changing the appearance of the skin by reducing near term redness, or reducing the development of darkening or tanning that would otherwise develop via interaction of the skin with ultraviolet light.

It is also known to apply agents that affect the appearance of the skin by penetrating the skin to some depth and interacting biologically with the skin and other tissues. For example, retinoids can affect skin appearance by interacting biologically with the body to enhance the formation of collagen and elastin, also potentially reducing the appearance of wrinkles in the skin.

It is also known that certain skin darkening agents such as dihydroxyacetone chemically react with skin proteins, causing the skin to take on a darker tone. Other skin darkening agents, essentially synthetic versions of MSH (melanin stimulating hormone) can darken the skin by, for example, stimulating the production of melanin, a dark pigment found in skin melanocytes. Working in the contrary manner, certain skin lightening or skin whitening agents such as hydroquinone and extracts of soy can affect skin appearance by reducing the production of tyrosinase, an enzyme which is needed for the skin to produce melanin.

One particular class of compounds known for skin-lightening or skin-whitening effects are alkyresorcinols. For example, it has been reported that purified hexylresorcinol is a suitable skin lightening or skin whitening agent (see US Patent Application Publication No. 2008/0305059 to Chaudhuri et al.).

Applicants have now unexpectedly discovered that topical application of skin-lightening resorcinols can actually promote the formation of such building blocks of the skin as elastin and collagen. Applicants have also found that skin-lightening resorcinols can actually quite unexpectedly be combined with skin *darkening* agents, thereby offering the opportunity to enhance the formation of elastin and collagen without necessarily causing what may be an undesirable lightening or whitening of the skin.

### SUMMARY OF THE INVENTION

The invention provides a composition comprising a skin-lightening resorcinol and a skin darkening agent as described in claim 1.

According to another aspect, the invention provides a cosmetic method of treating a sign of skin aging comprising topically applying to skin in need of such treatment a composition comprising a skin-lightening resorcinol and a skin darkening agent as described herein.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Unless otherwise indicated, a percentage or concentration refers to a percentage or concentration by weight (i.e., % (W/W). Unless stated otherwise, all ranges are inclusive of the endpoints, e.g., "from 4 to 9" includes the endpoints 4 and 9.

In certain embodiments, compositions of the present invention are suitable for darkening the skin. As used

herein, "darkening" is darkening the appearance of human skin or hair, including, but not limited to, darkening human skin to either achieve a "sun tan" effect or to cover light areas of the skin (e.g., as a result of a scar or a disease or a therapy) or darkening natural hair color or restoring discolored hair due to aging (e.g., gray or white hair) or external aggressions (e.g., excess exposure to sun or chlorine). While throughout the specification reference is made to darkening the skin, it is believed that other keratinous bodily materials such as hair may also be darkened.

Products described herein may optionally be in finished packaged form. In one embodiment, the package is a container such as a plastic, metal or glass tube or jar containing the composition. The product may further contain additional packaging such as a plastic or cardboard box for storing such container. In one embodiment, the product comprises a composition of the invention and contains instructions directing the user to apply the composition to the skin or hair to darken the skin (e.g., to tan the skin), even skin tone (e.g., to darken light areas of the skin or to treat or prevent mottled hyperpigmentation), darken the hair (e.g., to darken light brown, blonde, gray or white hairs), or treat one or more signs of skin aging as set forth below. Such instructions may be printed on the container, label insert, or on any additional packaging.

As used herein, "topically applying" means directly laying on or spreading on outer skin, the scalp, or hair, e.g., by use of the hands or an applicator such as a wipe, roller, or spray.

As used herein, "cosmetically acceptable" means that the ingredients the term describes are suitable for use in contact with tissues (e.g., the skin or hair) without undue toxicity, incompatibility, instability, irritation, allergic response, or the like.

As used herein, a "skin darkening effective amount" means an amount sufficient to induce a darkening of human skin or hair. This amount will vary with the area being treated, the age and skin or hair type of the end user, the duration and nature of the treatment, the specific composition employed, the carrier utilized, and like factors.

In certain embodiments, compositions of the present invention are suitable for treating signs of skin aging. As used herein, "signs of skin aging" includes the presence of lines and wrinkles, loss of elasticity, uneven skin, and blotchiness. In a particularly preferred embodiment, the sign of aging is the presence of lines and wrinkles.

As used herein, "treating signs of skin aging" refers to mitigating, reducing, preventing, improving, or eliminating the presence or signs of skin aging described above.

As used herein, "wrinkle" includes fine lines, fine wrinkles, or coarse wrinkles. Examples of wrinkles include, but are not limited to, fine lines around the eyes (e.g., "crow’s feet"), forehead and cheek wrinkles, frown-lines, and laugh-lines around the mouth.

As used herein, "loss of elasticity" includes loss of elasticity or structural integrity of the skin or tissue, including but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically effective amount" means an amount of a physiologically active compound or composition sufficient for treating one or more signs of skin aging, but low enough to avoid serious side effects. The cosmetically effective amount of the compound or composition will vary with the particular condition being treated, the age and physical condition of the end user, the severity of the condition being treated/prevented, the duration of the treatment, the nature of other treatments, the specific compound or product/composition employed, the particular cosmetically-acceptable carrier utilized, and like factors.

It is believed that one skilled in the art can, based upon the description herein, utilize the present invention to its fullest extent. The following specific embodiments are to be construed as merely illustrative, and not limitative of the remainder of the disclosure in any way whatsoever.

Skin-Lightening Resorcinol

Resorcinol is a dihydroxy phenol compound (i.e., 1,3-dihydroxybenzene), having the following structure:

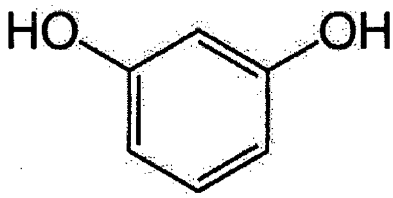

The skin-lightening resorcinols used herein are "substituted resorcinols" as described in claim 1.

The skin-lightening resorcinol is selected from 4-hexyl resorcinol and 4-octylresorcinol, particularly 4-hexyl resorcinol. The structures of 4-hexylresorcinol and 4-octylresorcinol are shown below:

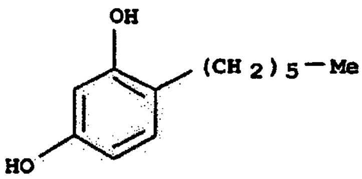

4-hexyl resorcinol

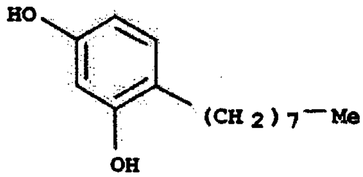

4-octylresorcinol

4-Hexyl resorcinol is commercially available as "SYNOVEA HR" from Sytheon of Lincoln Park, NJ. 4-Octylresorcinol is commercially available from City Chemical LLC of West Haven, Connecticut.

The skin-lightening resorcinol is present in the composition in a safe and effective amount, such as from 0.01% to 10%, preferably from 0.1% to 5%, more preferably from 0.2% to 2%, even more preferably from 0.5% to 1.5%, by weight of the composition.

Skin Darkening Agent

The compositions of the invention comprise a skin darkening agent as described in claim 1. As used herein, "skin darkening agent" means an organic moiety or molecule capable of darkening the skin when topically applied thereto, and in particular, an organic moiety or molecule that is capable of darkening the skin without the skin being exposed to UV-radiation.

In a preferred embodiment, the skin darkening agent is capable of providing sufficient darkening to the skin, so as to provide a negative change in darkness relative to a control, - % dL, that is at least 5%, preferably at least 15%, more preferably at least 30%, when tested according to the IN-VIVO SKIN DARKENING TEST evaluated at 3, 6 and/or 24 hours. The IN-VIVO SKIN DARKENING TEST is performed as follows. Two human subjects having Fitzpatrick Skin Types I-III, age ranging from 21-50 are used. For each subject, a location for each of the compositions to be tested is determined and initial chromometer X readings are taken there using a chromometer such as a CR-400 chromometer available from Konica Minolta Sensing of Ramsey, NJ. These readings are averaged to arrive at a baseline reading for each subject. A test sample is prepared by mixing a skin darkening agent in a suitable solvent in order to evenly dissolve or suspend the skin darkening agent. The solvent is preferably water, propylene glycol, or a mixture thereof, but if the skin darkening agent is difficult to dissolve or evenly distribute, up to 50% ethanol may be used, such as 50% ethanol/ 50% water. A control (no skin darkening agent, only solvent) and the test composition are each separately applied to a distinct area of the forearm for each subject at a coverage of 18 mg over 9 $cm^2$ of skin area and are rubbed in. The compositions are allowed to dry and after three time periods, 3 hours, 6 hours, and 24 hours, additional chromometer readings are taken. The change in lightness, dL, is calculated by subtracting the average reading after treatment to average reading of baseline. A change in lightness will typically be negative (indicating a darkening effect) effect) for a skin darkening agent.

The change in lightness for a test sample is normalized to that of the control. This normalized change in lightness is calculated as:

$$\% dL = 100 * (dL_{test\ sample} - dL_{control}) / dL_{control}$$

Typically, the change in lightness will be negative for application of a skin darkening agent. As such, in order to avoid using negative numbers, one may refer to the

normalized change in darkness = - % dL

The skin darkening agent is a melanin enhancement agent as described in claim 1, e.g., an agent suitable for enhancing either melanin synthesis (e.g., by enhancing tyrosinase) or for enhancing the transport of melanin to keratinocytes.

Suitable melanin enhancement agents may be selected based on the IN-VITRO SKIN-DARKENING TEST described in U.S. Patent Application Publication No. US2008/0249029 entitled "Methods For Treating Skin Pigmentation,". In the IN-VITRO SKIN-DARKENING TEST, an *in vitro* epidermal equivalent system is used. The epidermal equivalent system contains melanocytes. One epidermal equivalent system useful in performing this study is the MelanoDerm system, available commercially from MatTek (Ashland, MA). This system contains human normal melanocytes, together with normal, human-derived epidermal keratinocytes, which have been cultured to form a multi layered, highly differentiated model of the human epidermis. To demonstrate an increase in pigmentation, equivalents are treated with test compounds, in the absence of ultraviolet light or solar exposure, for three days and samples are harvested on the fourth day after beginning of treatment. The harvested equivalents are then stained with DOPA (a substrate for tyrosinase) and H&E (a standard histological stain) or with Fontana-Mason (F&M) staining, another stain known to those of skill in the art. At least three sections sections per equivalent, three equivalents per experiment are processed. F&M staining is a silver staining technique that clearly and cleanly marks melanins which have high silver nitrate reducing activity. F&M stained sections can be used for image analysis using Optomax Image Analysis Systems, from Optomax Inc. (Hollis, N.H) used on a Gateway 2000 PS-100 computer (Media Cybernetics, Silver Springs, Md.) for capturing images. Image Pro Plus version 4.0 can be used for image analysis. Parameters that are measured include: (1) level of pigmentation within individual melanocytes and (2) number of pigmented melanocytes per field, for the Optomax system, or (1) the surface area of silver deposits within melanocytes and (2) the number of pigmented melanocytes for the Image Pro system. Using the Optomax system, surface area of silver deposits within individual melanocytes can be measured in 60 melanocytes, using multiple sections from triplicate equivalents per treatment. The number of melanocytes per field can be calculated in these sections. A "pigmentation factor" is defined as the average surface area of silver deposits within an individual melanocyte, multiplied by the number of pigmented melanocytes per field. A value of one is assigned to untreated controls, and values of treatment groups are normalized to their relevant controls. Suitable melanin enhancement agents have a pigmentation factor of at least 1.2, preferably at least 1.5, and most preferably at least 2.

The melanin enhancement agents in the present invention are amine compounds of formula I, shown below:

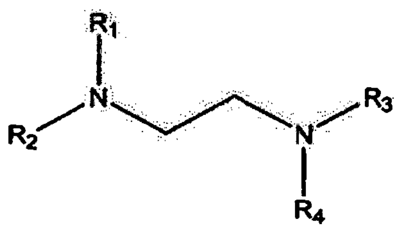

(I)

wherein $R_1$, $R_2$, $R_3$ and $R_4$ are independently selected from the group consisting of hydrogen, $C_1$-$C_6$ alkyl, and $C_1$-$C_6$ hydroxyalkyl; or a cosmetically-acceptable salt thereof. Examples of preferred amine compounds of formula I include, but are not limited to, N,N,N',N'-tetrakis(2-hydroxypropyl)ethylenediamine (THPED), N, N, N', N'-tetrakis (2-hydroxyethyl) ethylene diamine (THEED), and N, N, N', N'-tetramethylethylene diamine (TEMED), the structures of which are set forth below, enantiomers thereof, diastereoisomers thereof, or cosmetically-acceptable salts thereof.

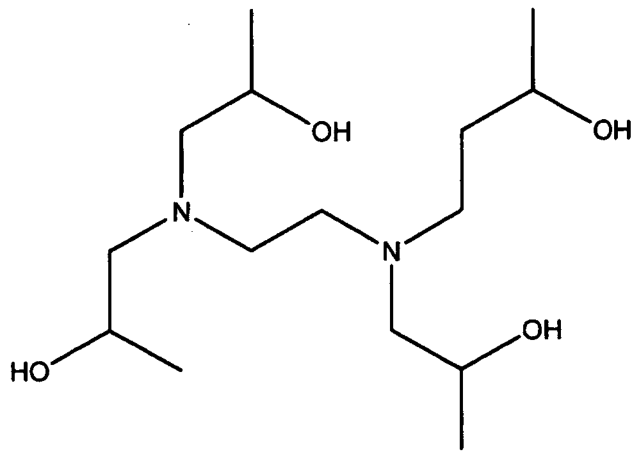

(THPED)

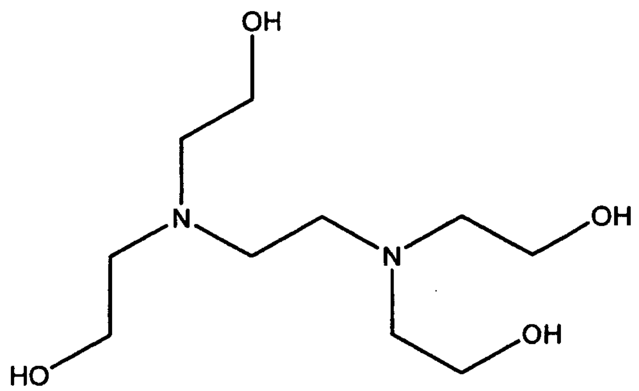

(THEED)

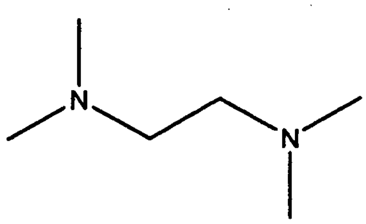

(TEMED)

The synthesis of N,N,N',N'-tetrakis (2-hydroxypropyl) ethylenediamine from the reaction of ethylenediamine with propylene oxide is described in U.S. Patent No. 2,697,118.

The skin darkening agent is present in the composition in a safe and effective amount. For example, the amount of skin darkening agent in the compositions of the invention may range from, on an individual basis or in combination with one another, about 0.01 % to about 10%, preferably from about 0.1 % to about 5%, more preferably from about 0.2% to about 2%, and even more preferably from about 0.5% to about 2%, by weight of the composition.

The ratio of concentration of skin darkening agent to concentration of skin-lightening resorcinol may be varied according to the desired effectiveness of the composition in treating of signs of aging and optionally darkening the skin. For example, the skin darkening agent and skin-lightening resorcinol may be present in a concentration by weight ratio (which is determined by dividing the concentration by weight of the skin darkening agent by the concentration by weight

of the skin-lightening resorcinol) of at least about 0.1, preferably at least about 0.25, more preferably from about 0.25 to about 10, such as from about 0.5 to about 5, from about 0.75 to about 5, and even more particularly from about 1.25 to about 5.

Topical Compositions

The compositions of the present invention are applied topically to human skin or hair. In addition to the skin-lightening resorcinol and the skin darkening agent and the optional darkening enhancement ingredients, the composition may further include a cosmetically acceptable topical carrier that may be from 50% to 99.99%, by weight, of the composition (e.g., from 80% to 99%, by weight, of the composition). In a preferred embodiment of the invention, the cosmetically acceptable topical carrier includes water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The compositions useful in the present invention can be formulated as solutions. Solutions typically include an aqueous or organic solvent (e.g., from 50% to 99.99% or from 90% to 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

Compositions useful in the subject invention may be formulated as a solution comprising an emollient. Such compositions preferably contain from 2% to 50% of an emollient(s). As used herein, "emollients" refer to materials used for the prevention or relief of dryness, such as by preventing the transepidermal loss of water from the skin. Examples of emollients include, but are not limited to, those set forth in the International Cosmetic Ingredient Dictionary and Handbook, eds. Pepe, Wenninger and McEwen, pp. 2930-36 (The Cosmetic, Toiletry, and Fragrance Assoc., Washington, D.C., 9th Edition, 2002) (hereinafter "ICI Handbook"). Examples of particularly suitable emollients include vegetable oils, mineral oils, fatty esters, and the like.

A lotion can be made from such a solution. Lotions typically contain from 1% to 20% (e.g., from 5% to 10%) of an emollient(s) and from 50% to 90% (e.g., from 60% to 80%) of water.

Another type of product that may be formulated from a solution is a cream. A cream typically contains from 5% to 50% (e.g., from 10% to 20%) of an emollient(s) and from 45% to 85% (e.g., from 50% to 75%) of water.

Although it is preferred that the composition of the present invention includes water, the composition may alternatively be anhydrous or an ointment that includes no water but organic and/or silicone solvents, oils, lipids and waxes. An ointment may contain a simple base of animal or vegetable oils or semi-solid hydrocarbons. An ointment may contain from 2% to 10% of an emollient(s) plus from 0.1% to 2% of a thickening agent(s). Examples of thickening agents include, but are not limited to, those set forth in the ICI Handbook pp. 2979-84.

The composition may be formulated as an emulsion. If the topical carrier is an emulsion, from 1% to 10% (e.g., from 2% to 5%) of the topical carrier contains an emulsifier(s). Emulsifiers may be nonionic, anionic or cationic. Examples of emulsifiers include, but are not limited to, those set forth in the ICI Handbook, pp.2962-71.

Lotions and creams can be formulated as emulsions. Typically such lotions contain from 0.5% to 5% of an emulsifier(s). Such creams typically contain from 1% to 20% (e.g., from 5% to 10%) of an emollient(s); from 20% to 80% (e.g., from 30% to 70%) of water; and from 1% to 10% (e.g., from 2% to 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between 0.1% and 5%, by weight, of such gelling agents.

The compositions of the present invention can also be formulated into a solid formulation (e.g., a wax-based stick, soap bar composition, powder, or a wipe containing powder).

The compositions useful in the subject invention may contain, in addition to the aforementioned components, a wide variety of additional oil-soluble materials and/or water-soluble materials conventionally used in compositions for

use on skin and hair, at their art-established levels.

Additional Cosmetically Active Agents

In one embodiment, the composition further contains another cosmetically active agent. As used herein, a "cosmetically active agent" is a compound (e.g., a synthetic compound or a compound isolated from a natural source or a natural extract) that has a cosmetic or therapeutic effect on the skin or hair, including, but not limiting to, anti-acne agents, shine control agents, anti-microbial agents, anti-inflammatory agents, anti-mycotic agents, anti-parasite agents, external analgesics, sunscreens, photoprotectors, antioxidants, keratolytic agents, surfactants, moisturizers, nutrients, vitamins, energy enhancers, anti-perspiration agents, astringents, deodorants, firming agents, anti-callous agents, and agents for hair and/or skin conditioning.

In one embodiment, the agent is selected from, but not limited to, the group consisting of hydroxy acids, benzoyl peroxide, D-panthenol, octyl methoxycinnimate, octyl salicylate, homosalate, avobenzone, carotenoids, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides, amino acids such as proline, vitamins, lactobionic acid, acetyl-coenzyme A, niacin, riboflavin, thiamin, ribose, electron transporters such as NADH and FADH2, and other botanical extracts such as aloe vera, feverfew, oatmeal and derivatives and mixtures thereof. The cosmetically active agent will typically be present in the composition of the invention in an amount of from 0.001% to 20% by weight of the composition, e.g., 0.005% to 10% such as 0.01% to 5%.

Examples of vitamins include, but are not limited to, vitamin A, vitamin Bs such as vitamin B3, vitamin B5, and vitamin B12, vitamin C, vitamin K, and different forms of vitamin E like alpha, beta, gamma or delta tocopherols or their mixtures, and derivatives thereof.

Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis.

Other Materials

Various other materials may also be present in the composition, as known in the art. These include humectants, pH adjusters, chelating agents (e.g., EDTA), fragrances, dyes, and preservatives (e.g., parabens).

The composition and formulations and products containing such compositions of the present invention may be prepared using methodology that is well known by an artisan of ordinary skill.

Methods of Use

Compositions of the present invention may be topically applied to mammalian skin, such as skin that is in need of treatment for one or more signs of skin aging as described above. In one embodiment, the compositions are applied to skin in need of treatment for lines and wrinkles and/or loss of elasticity. Compositions of the present may also be applied to skin that is in need of increased pigmentation. The compositions may be applied to the skin in need of such treatment according to a suitable treatment regimen, e.g., every month, every week, every other day, every day, twice a day, or the like.

The following non-limiting example further illustrates the invention.

Example 1: Preparation of Inventive Example, Ex. 1

The following skin darkening base, Composition A, was prepared:

| TRADE NAME | INGREDIENT | Wt. % |
|---|---|---|
| **Phase A** | | |
| | Water | 77.55 |
| Ultrez 10 | Carbomer | 0.75 |
| Versene NA | Disodium EDTA | 0.2 |
| **Phase B** | | |
| Brij 72 | Steareth-2 | 0.75 |
| Brij 721 | Steareth-21 | 1.50 |
| Finsolv TN | C12-15 Alkyl Benzoate | 2.00 |
| Dow Coming Q7 9120 Fluid | Dimethicone | 5.000 |
| **Phase C** | | |
| Penonip XB | Preservative | 1.00 |
| Neutrol TE | Tetrahydroxypropyl ethylenediamine | 1.25 |
| **Phase D** | | |
| | Water | 8.5 |
| Neutrol TE | Tetrahydroxypropyl ethylenediamine | 1.25 |
| Citric Acid | Citric Acid | 0.25 |
| | | |
| TOTAL | | 100.00 |

Composition A was blended with the following additional ingredients to produce a composition according to the invention:

| TRADE NAME | INGREDIENT | Wt. % |
|---|---|---|
| Composition A | | 70.0 |
| Transcutol CG | | 5.00 |
| Citric acid, 1% | Citric acid | 0.01 |
| Versene NA | Disodium EDTA | 0.01 |
| Synovea HR | 4-hexylresorcinol | 1.00 |
| Butylene glycol | Butylene glycol | 20.0 |
| Water | Water | 3.98 |
| | | |
| TOTAL | | 100.0 |

This composition comprised a skin darkening agent, specifically tetrahydroxypropyl ethylenediamine, and a skin-lightening resorcinol, specifically, 4-hexylresorcinol. The concentration by weight ratio of skin darkening agent to skin-lightening resorcinol in the composition was (0.7 X 2.5%)/1.0 % = 1.75.

The composition was placed at room temperature as well as at elevated temperature (50°C) for 7 days. No phase instabilities were observed.

## Claims

**1.** A composition comprising:

a skin-lightening resorcinol, wherein the skin-lightening resorcinol is selected from 4-hexyl resorcinol and 4-octyl resorcinol; and

a skin darkening agent, wherein the skin darkening agent is an amine compound of formula I:

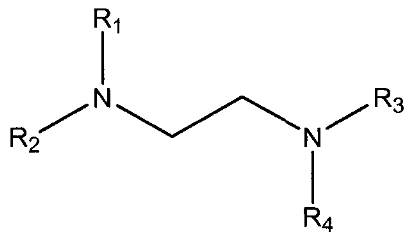

(I)

wherein $R_1$, $R_2$, $R_3$, and $R_4$, are independently selected from the group consisting of hydrogen, $C_1$-$C_6$ alkyl, and $C_1$-$C_6$ hydroxyalkyl; or a cosmetically-acceptable salt thereof.

**2.** The composition of claim 1, wherein the skin-lightening resorcinol is 4-hexylresorcinol.

**3.** The composition of any one of claims 1 to 2, wherein the skin darkening agent is selected from the group consisting ofN,N,N',N'-tetrakis(2-hydroxypropyl)ethylenediamine, N, N, N', N'-tetrakis (2-hydroxyethyl) ethylene diamine, N, N, N', N'-tetramethylethylene diamine, enantiomers thereof, diastereoisomers thereof, and cosmetically-acceptable salts thereof.

**4.** The composition of any preceding claim, wherein the skin darkening agent and the skin-lightening resorcinol are present in a concentration by weight ratio of at least 0.1.

**5.** A cosmetic method of treating a sign of aging, comprising topically applying to skin in need of said treatment a composition as claimed in any one of claims 1 to 4.

**6.** The cosmetic method of claim 5, wherein the sign of aging is selected from the group consisting of presence of lines and wrinkles and loss of elasticity.

**Patentansprüche**

**1.** Zusammensetzung, die Folgendes umfasst:

ein hautaufhellendes Resorcinol, wobei das hautaufhellende Resorcinol aus 4-Hexylresorcinol und 4-Octylresorcinol ausgewählt ist; und

ein Hauttönungsmittel, wobei es sich bei dem Hauttönungsmittel um eine Aminverbindung der Formel I:

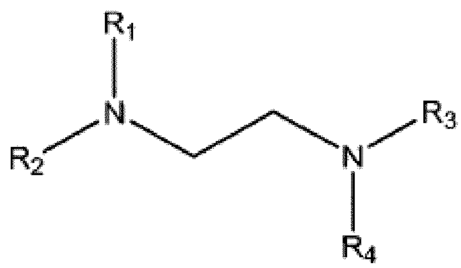

(I)

worin $R_1$, $R_2$, $R_3$ und $R_4$ unabhängig aus der Gruppe bestehend aus Wasserstoff, $C_1$-$C_6$-Alkyl und $C_1$-$C_6$-Hydroxyalkyl ausgewählt sind, oder um ein kosmetisch unbedenkliches Salz davon handelt.

**2.** Zusammensetzung nach Anspruch 1, wobei es sich bei dem hautaufhellenden Resorcinol um 4-Hexylresorcinol handelt.

**3.** Zusammensetzung nach einem der Ansprüche 1 bis 2, wobei das Hauttönungsmittel aus der Gruppe N,N,N',N'-Tetrakis(2-hydroxypropyl)ethylendiamin, N,N,N',N'-Tetrakis(2-hydroxyethyl)ethylendiamin, N,N,N',N'-Tetramethylethylendiamin, deren Enantiomeren, deren Diastereomeren und kosmetischen unbedenklichen Salzen davon ausgewählt ist.

**4.** Zusammensetzung nach einem vorhergehenden Anspruch, wobei das Hauttönungsmittel und das hautaufhellende Resorcinol in einer Konzentration von einem Gewichtsverhältnis von mindestens 0,1 vorliegen.

**5.** Kosmetikmethode zum Behandeln einer Alterungserscheinung, bei dem eine Zusammensetzung nach einem der Ansprüche 1 bis 4 topisch auf Haut, die dieser Behandlung bedarf, aufgetragen wird.

**6.** Kosmetikmethode nach Anspruch 5, wobei die Alterungserscheinung aus der Gruppe bestehend aus dem Vorliegen von Falten und Fältchen und Elastizitätsverlust ausgewählt ist.

## Revendications

**1.** Composition comprenant :

un résorcinol pour éclaircir la peau, **caractérisé en ce que** le résorcinol pour éclaircir la peau est choisi parmi le 4-hexylrésorcinol et le 4-octylrésorcinol ; et
un agent pour foncer la peau, **caractérisé en ce que** l'agent pour foncer la peau est un composé d'amine de formule I :

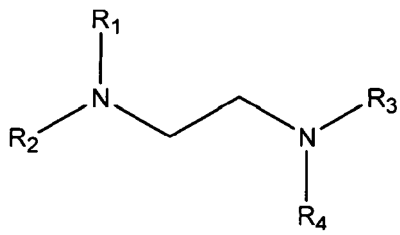

(I)

dans laquelle $R_1$, $R_2$, $R_3$ et $R_4$ sont choisis indépendamment dans le groupe constitué par hydrogène, $C_1$-$C_6$alkyle et $C_1$-$C_6$hydroxyalkyle ; ou un sel cosmétiquement acceptable de celui-ci.

**2.** Composition selon la revendication 1, **caractérisée en ce que** le résorcinol pour éclaircir la peau est le 4-hexylrésorcinol.

**3.** Composition selon l'une quelconque des revendications 1 à 2, **caractérisée en ce que** l'agent pour foncer la peau est choisi dans le groupe constitué par la N,N,N',N'-tétrakis(2-hydroxypropyl)éthylène-diamine, la N,N,N',N'-tétrakis (2-hydroxyéthyl)-éthylènediamine, la N,N,N',N'-tétraméthyléthylène-diamine, des énantiomères ce celles-ci, des diastéréoisomères de celles-ci, et des sels cosmétiquement acceptables de celles-ci.

**4.** Composition selon l'une quelconque des revendications précédentes, **caractérisée en ce que** l'agent pour foncer la peau et le résorcinol pour éclaircir la peau sont présents selon une concentration en rapport pondéral d'au moins 0,1.

**5.** Méthode cosmétique destinée à traiter un signe de vieillissement, comprenant l'application topique à une peau ayant besoin dudit traitement, d'une composition selon l'une quelconque des revendications 1 à 4.

**6.** Méthode cosmétique selon la revendication 5, **caractérisée en ce que** le signe de vieillissement est choisi dans le groupe constitué par la présence de ridules et de rides et la perte d'élasticité.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080305059 A, Chaudhuri
- US 20080249029 A
- US 2697118 A


### Non-patent literature cited in the description


- International Cosmetic Ingredient Dictionary and Handbook. The Cosmetic, Toiletry, and Fragrance Assoc, 2002, 2930-36